# EUROPEAN PATENT APPLICATION

(11) **EP 2 382 974 A1**
(43) Date of publication of application: **02.11.2011**
(21) Application number: 09831506.2
(22) Date of filing: 09.12.2009
(51) Int. Cl.: A61K 31/395, A61P 35/00

(54) **USE OF CYCLOSQUARAMIDE COMPOUNDS AS ANTI-TUMOUR AGENTS**

(30) Priority: 10.12.2008 ES 200803496
(71) Applicant: Universitat de les Illes Balears, Palma de Mallorca 07122 (ES)
(72) Inventor: COSTA TORRES, Antonio, E-07122 Palma de Mallorca (ES); ROTGER PONS, María del Carmen, E-07122 Palma de Mallorca (ES); FERNÁNDEZ DE MATTOS, Silvia, E-07122 Palma de Mallorca (ES); DE VILLALONGA SMITH, Priam, E-07122 Palma de Mallorca (ES)
(74) Representative: Pons Ariño, Angel
(86) International application number: PCT/ES2009/070565
(87) International publication number: WO 2010/066933

(57) **Abstract**

Use of squaramide-based macrocylic compounds as kinase inhibitors and as anti-tumour agents. More specifically, said compounds are used to treat diseases such as cancer, diabetes, neurodegenerative diseases, HIV replication, etc. Furthermore, the present invention likewise relates to pharmaceutical compositions that contain said compounds

## Description

The present invention relates to the use of squaramide-based macrocylic compounds, as inhibiting agents of a series of kinases and as anti-tumour agents. The kinases inhibited are enzymes highly relevant for cell proliferation and transformation and are related to diseases such as cancer, diabetes, neurodegenerative diseases, HIV replication etc. Furthermore, the present invention likewise relates to pharmaceutical compositions that contain said compounds.

### STATE OF THE PRIOR ART

The term cancer includes an extensive set (>100) of different pathological entities, whose common denominator is uncontrolled cell growth, as a consequence of characteristic genetic alterations of the tumour cell. The altered genes (oncogenes and tumour suppressors) send wrong messages or messages different to those they should send. As a consequence, the cell acquires the capacity of multiplying itself autonomously and uncontrollably causing the development of tumours, which also acquire progressively more malignant characteristics such as angiogenesis and metastasis or remote dissemination. Thus, the mutation of an oncogene such as K-Ras, very frequent in cancerous cells, induces the hyperactivation of certain cell signalling pathways, which transfer information to the nucleus where they activate the programs responsible for cell division and survival, among other processes. These alterations may constitute the therapeutic target with the aim of inhibiting cell proliferation, inducing apoptosis or both.

Many of the alterations that take place in the malignant cells are linked to receptors involved with enzymatic activity. There are four classes of enzymes linked to receptors of this type: guanylyl cyclases, tyrosine phosphatases, serine/threonine kinases and protein-tyrosine kinases. The last three play a very important role in malignant cell transformation.

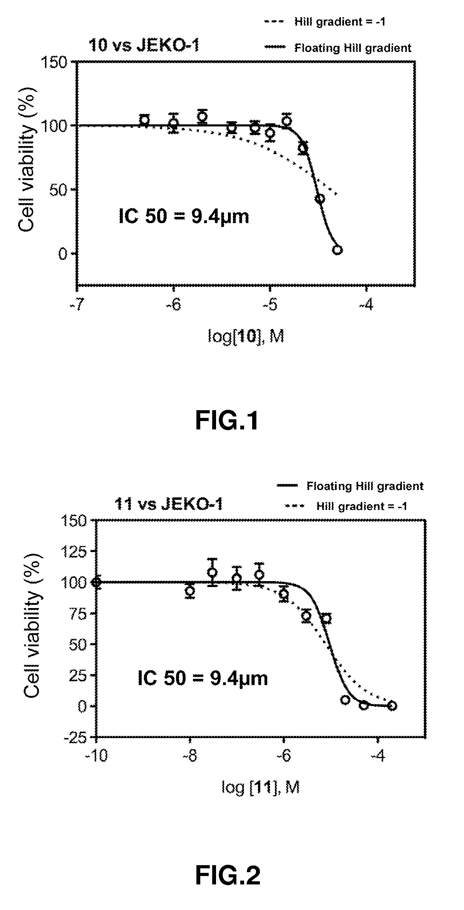

Cell signalling in the interior of the malignant cells may also be inhibited by external agents. Given that the abnormal regulation of kinases and phosphatases seems to directly contribute to the appearance of a good number of cancers, there is great interest in the search for selective kinase inhibitors with two purposes: first, blocking the cell cycle andstopping cell proliferation, thereby consisting of anti-tumour agents *per se,* and second, inhibiting the effect of the cell cycle checkpoints (G1, S and G2) avoiding the correction of DNA damage caused by classic anti-tumour agents and thus avoiding the mechanisms of cell resistance to the action of anti-tumour agents (figure 1). This latter enables a drastic reduction of the dose of conventional cytotoxic agents without losing the efficacy of the treatment and, therefore, reducing its secondary effects.

In this line, it has been demonstrated that tyrosine kinase inhibitors are useful as selective inhibitors of cancer cell growth in mammals. For example, Gleevec ^{™}, also known as imatinib mesylate, or STI571, inhibits among others the activity of the kinase product of the fusion of the BCR-ABL genes and has been approved for treatment of CML. There are some kinase inhibitors that show even greater selectivity such as, Tarceva^{™} which only inhibits the EGF receptor kinase with high potential although it can also inhibit the transduction signal of other kinase receptors, probably due to the fact that these receptors heterodimerize with the EGF receptor.

In general, traditional anti-tumour agents (alkylating agents, antimetabolites, cis-platin derivatives, etc.) are cytotoxic, which entails a lack of selectivity and causes the appearance of adverse secondary effects. In parallel with our increasing understanding of the mechanismsgoverning tumour progression, cancer therapy is evolving intelligently towards the development of therapeutic agents that act specifically against the cancer cells by the development of agents that inhibit cell proliferation and survival, the so-called target drugs.

Although compounds such as the aforementioned have considerably contributed to the development of anti-tumour agents, there is a continuous need to obtain better anticancer drugs and it is especially desirable to develop new compounds with better selectivity or potency, in addition to also reducing their secondary effects and toxicity.

The squaramides considered vinylogous to amides are a very suitable structural unit for preparing compounds mimetic to proteins and have a great capacity to intervene in the formation of hydrogen bonds and dynamic properties favourable for the establishment of secondary structures. This is based on the fact that a disecondary squaramide offers two hydrogen bond donor oxygen groups and two NH acceptor groups, arranged so that they can synergically act to establish favourable secondary interactions.

Compounds based on (arylamidoaryl) squaramide and indazole squaramide type structures have proven to be potent inhibitors of kinase receptors of the type c-Kit and CHK1, CHK2, SGK, respectively, which gives them an interesting anti-tumour activity.

Furthermore, macrocycles are structures abundant in nature and their potential uses are unlimited, making them the subject of intense research (Driggers, E.M., et al., Nature reviews. Drug discovery, Jul. 2008, 7(7), 608-624). These ring-type structures generally formed by C, N and O have been, for the last 50 years, the basis of many leading compounds in pharmacological studies, serving as inspiration for almost every new drug introduced by the pharmaceutical industry in the market, such as the example of cyclosporine and vancomycin among others. The macrocyclic structure is particularly attractive for the pharmaceutical industry since the ring-type structure helps to stabilize the drug from the biodegradation they suffer in the human body and increases efficacy, fixing the biologically active formation.

### DESCRIPTION OF THE INVENTION

The present invention provides the use of squaramide-based macrocylic compounds, as kinase inhibitor agents and anti-tumour agents. The kinases inhibited are enzymes highly relevant for cell proliferation and transformation and are related to diseases such as cancer, diabetes, neurodegenerative diseases, HIV replication, among others. The results obtained (see examples) demonstrate the use of squaramide macrocycles in the development of drugs indicated for the treatment of various types of cancer, such as B and T cell lymphoma or glioblastoma.

The macrocyclic compounds of the present invention of squaramide base and various sizes are potent "in vitro" kinase inhibitors. Furthermore, it is demonstrated (see examples) that they have a cytotoxic and cytostatic activity to human tumour lines of lymphoma and glioblastoma.

The macrocycles of the invention can be obtained from a macrocyclization reaction of the respective oligosquaramide precursors. They are secondary squaramides bonded together by spacers of various nature and functionalized, in all cases or in some, with hydrogen bonds donor and/or acceptor groups.. The number of squaramide units in the macrocycles can vary from 2 to 10.

The hydrogen bond donor-acceptor capacity of the squaramides allows the synthesis of the macrocyclic compounds from preorganized oligosquaramide compounds, by a simple macrocyclization reaction, obtaining excellent yields in all cases (>80%).

A first aspect of the present invention relates to the use of the compounds of general formula (I) or any of their salts (hereinafter compounds of the invention) for the preparation of a pharmaceutical composition: 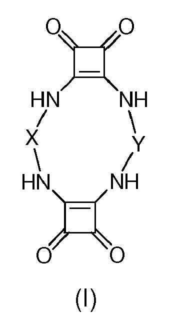 where: X and Y, are the same or different and selected from the list comprising:
- an alkyl group (C₁-C₁₀);
- a -R¹-NR-R²- group; or
- a -(R¹-NR-R²-squaramide- R¹-NR- R²)ₙ group; where
R is selected from the alkyl (C₁-C₁₀), aryl or heteroaryl groups, substituted or non-substituted;
R¹ and R² are the same or different and are selected from the alkyl (C₁-C₅) or acyl groups, substituted or non-substituted; and
n takes the values of 1 to 8, preferably n takes the values of 1 to 6.
"Squaramide" is understood to be the group of formula:

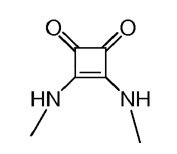

The term "alkyl" relates in the present invention to aliphatic chains, linear or branched, which have from 1 to 10 carbon atoms, for example, methyl, ethyl, n-propyl, i-propyl, n-butyl, tert-butyl, sec-butyl, n-pentyl, etc. The alkyl radicals can be optionally substituted by one or more substituents such as a cycloalkyl, aryl, heteroaryl, alkoxyl, halogen, nitro, amine or ammonium. Preferably it is a benzyl group, which can also be optionally substituted by one or more substituents such as alkoxyl, halogen, nitro, amino or ammonium.

The term "aryl" relates in the present invention to an aromatic carbocyclic chain, which has from 6 to 18 carbon atoms, and can be a single or multiple ring, in this last case with separated and/or condensed rings. A non-limiting example of aryl is a phenyl, naphthyl, indenyl group, etc... Preferably the aryl group is a phenyl.

The term "heteroaryl" relates in the present invention to an aromatic cyclic chain which has from 5 to 18 atoms, including from 1 to 5 heteroatoms, mainly N but it may contain O and/or S. The chain may be single or multiple ring. As non-limiting examples of heteroaryl we can cite azine, pyrrole, ozazole, pyridine, pirimidine, diazine, purine, etc

The term "acyl" relates, in the present invention, to a derivative of carboxylic acid due to elimination of a hydroxyl group. The derivatives of carboxylic acid have as general formula R³-CO-, where R³ is an alkyl group with the above exceptions and preferably relates to alkyl groups (C₁-C₇), linear or branched. R² forms an amide group with the nitrogen of the -R¹NRR²- group, and may be optionally substituted by one or more substituents such as an alkyl, cicloalkyl, heterocycloalkyl, aryl, heteroaryl, alkoxyl, halogen, haloalkyl, nitro, amino, alkyl ammonium, cycloalkyl ammonium or thiol. Preferably, R³ may be substituted by a thiol group which may be, in turn, substituted by an alkyl group.

In a preferred embodiment of the compounds of the invention, X or Y is a heptyl group.

When X or Y is selected from the -R¹NRR²- group or the -(R¹-NR- R²-squaramide- R¹-NR-R²)n group, preferably R¹ or R² is a propyl group or an acyl group (COR³), where R³ is an alkyl group, preferably a methyl group, substituted by a thioalkyl group (S(C₁-C₃)), preferably a thiomethyl group. In a preferred embodiment R is a methyl, benzyl or substituted benzyl group. When R is a substituted benzyl group it is preferably by a nitro group forming a nitrobenzyl group.

The compounds of general formula (I) can be selected from the following formulas from 1 to 11:

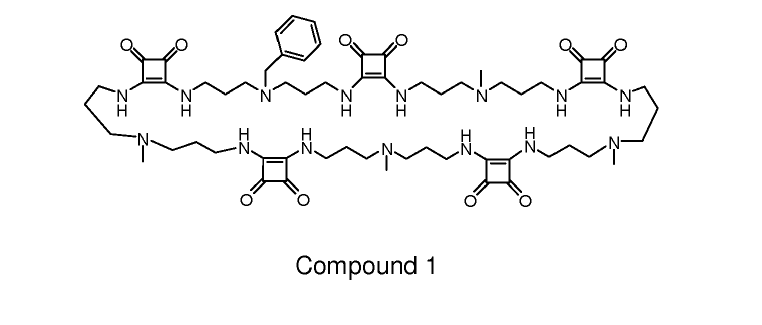

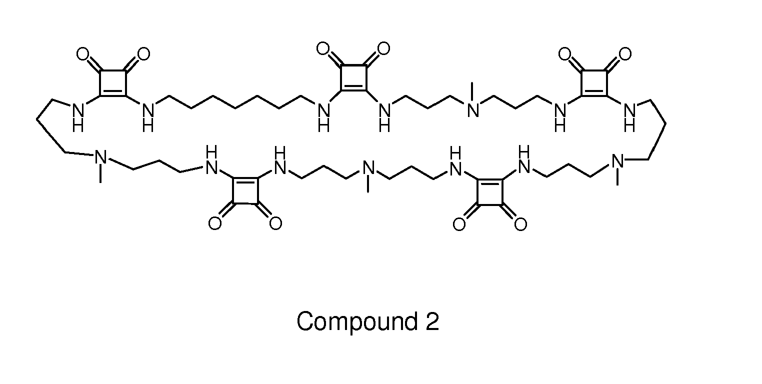

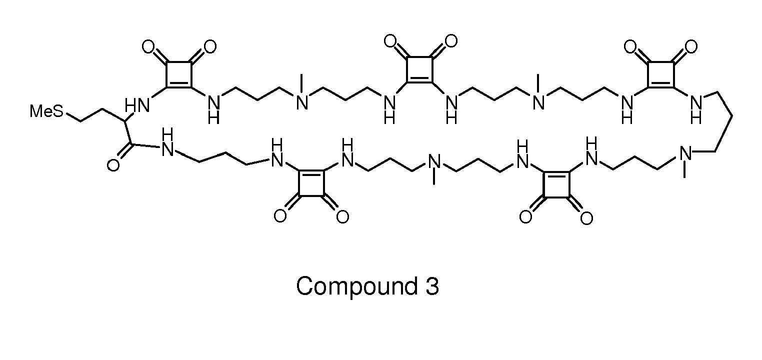

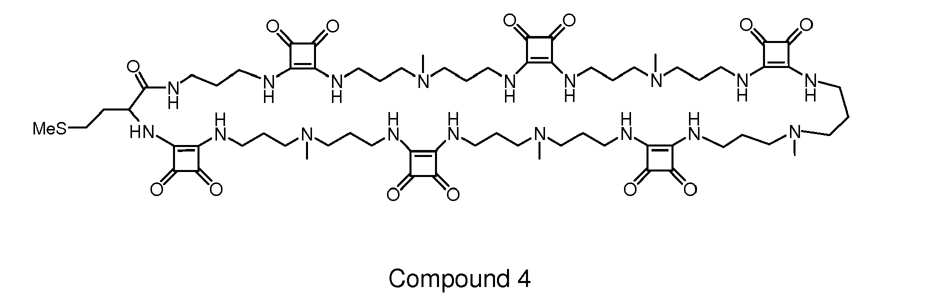

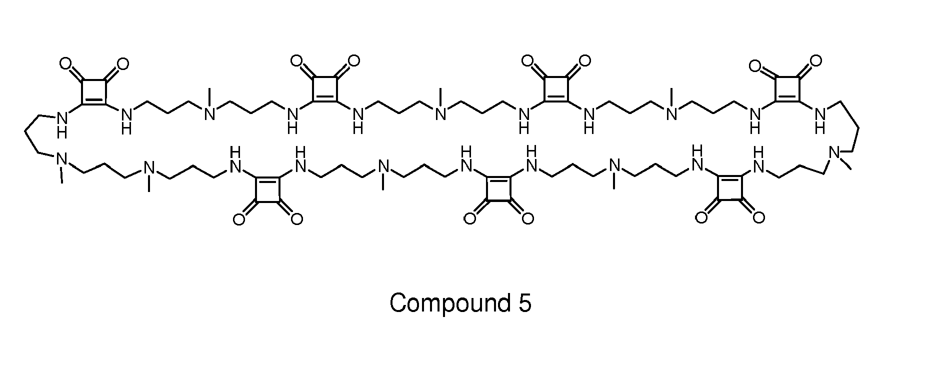

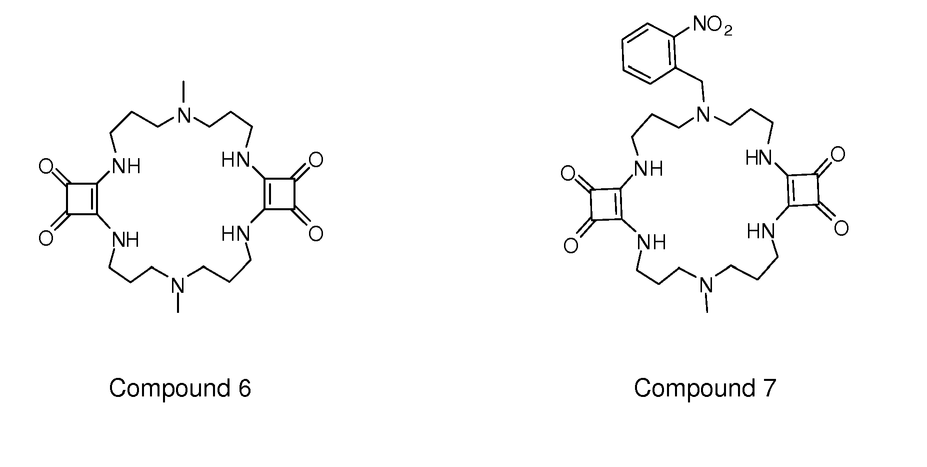

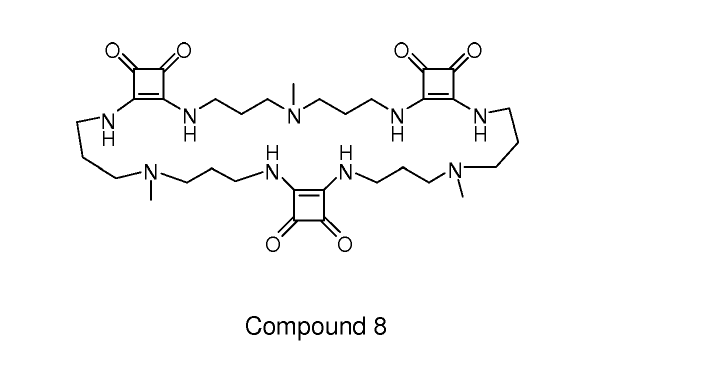

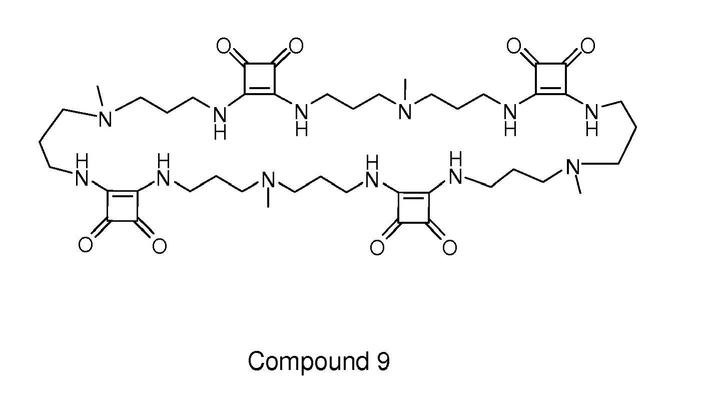

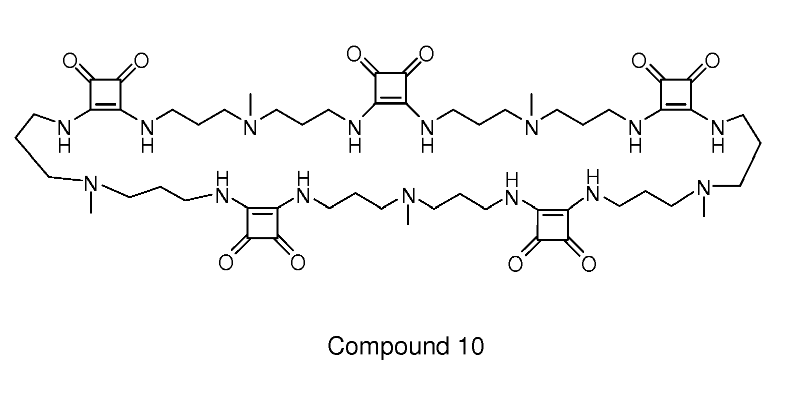

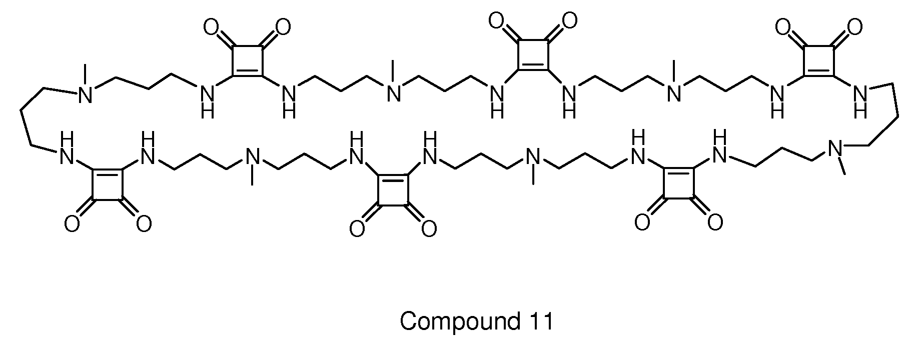

Another aspect of the present invention relates to the use of the compounds of the invention for the preparation of a pharmaceutical composition for the treatment of diseases associated with kinase inhibition.

The diseases associated with kinase inhibition can be selected from tumoral diseases, diabetes, neurodegenerative diseases (such as, for example Alzheimer's) or HIV replication.

Among the tumour types, the following stand out without excluding other types: B and T-cell lymphomas and glioblastoma.

Another aspect of the present invention relates to pharmaceutical composition comprising at least one compound of general formula (I) together with a pharmaceutically acceptable vehicle. Sad composition will be used in a therapeutically effective quantity.

The adjuvants and pharmaceutically acceptable vehicles that may be used in said compositions are the adjuvants and vehicles known by persons skilled in the art and typically used in the preparation of therapeutic compositions.

The compounds of the present invention, their pharmaceutically acceptable salts, prodrugs and/or solvates as well as the pharmaceutical compositions that contain them may be used together with other additional drugs, or active principles, to provide a combination therapy. Said additional drugs may form part of the same pharmaceutical composition or, alternatively, they may be provided in the form of a separate composition for their simultaneous administration or not to that of the pharmaceutical composition comprising a compound of formula (I), or a pharmaceutically acceptable prodrug, solvate, derivative or a salt thereof.

In the sense used in this description, the expression "therapeutically effective quantity" relates to the quantity of the agent or compound capable of developing the therapeutic action determined by their pharmacological properties, calculated to produce the desired effect and, in general, it will be determined, among other causes, by the typical characteristics of the compounds, including the age, condition of the patient, the severity of the alteration or disorder and the route and frequency of administration.

Said therapeutic composition can be prepared in the form of a solid form or aqueous suspension, in a pharmaceutically acceptable diluent. The therapeutic composition provided by the present invention may be administered by any appropriate administration route; to this end, said composition will be formulated in the suitable pharmaceutical form for the selected administration route. In a particular embodiment, the administration of the therapeutic composition provided by this invention is performed by oral, topical rectal or parenteral route (including subcutaneous, intraperitoneal, intradermal, intravenous, etc.). A review of the different pharmaceutical forms for drug administration and the necessary excipients to obtain them may be found, for example, in "Tratado de Farmacia Galénica", C. Faulí i Trillo, 1993, Luzán 5, S.A. Ediciones, Madrid, or in other typical or similar ones of the Spanish and US Pharmacopoeias.

Another aspect of the present invention relates to compounds 1 to 5, described above.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following figures and examples are provided by way of illustration, and are not intended to limit the present invention.

### BRIEF DESCRIPTION OF THE FIGURES

**Fig. 1****.** Represents cell viability (Jeko-1 cells) depending on the concentration of compound 10, with the aim of calculating the IC₅₀.
**Fig. 2****.** Represents cell viability (Jeko-1 cells) depending on the concentration of compound 11, with the aim of calculating the IC₅₀.
**Fig. 3****.** Flow cytometric profiles of Jeko-1 cells treated with compound 10 for the indicated time and stained with propidium iodide..
**Fig. 4****.** Flow cytometric profiles of JVM-2 cells treated with compound 10 for the indicated time and stained with propidium iodide. Fig. 5. Representative images of U87 control cells and U87 cells treated with compound 10. The graphic represents the percentage quantification of the formation of multicellular tumour spheroids in both conditions.

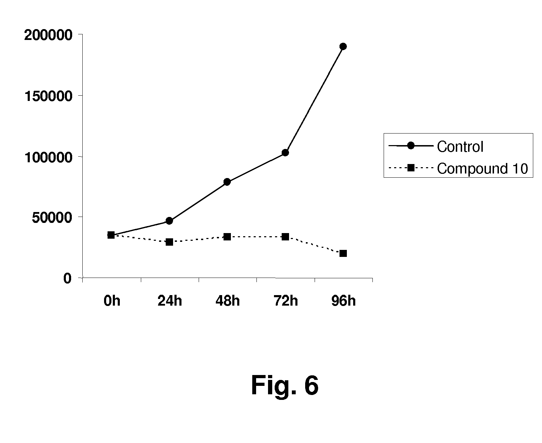

**Fig. 6****.** Representation of cell growth assays. The graphic represents the cell growth (increase in the number of cells/day) of untreated U87 cells (control) or treated with compound 10.

### EXAMPLES

Below, the invention will be illustrated by tests carried out by the inventors, which reveals the specificity and efficacy of the compounds of the invention.

### SYNTHESIS OF THE COMPOUNDS OF THE INVENTION

The macrocyclization reaction, as well as the synthesis and description of compounds 9 to 11, are described in the article by Rotger, C, et al., Angew. Chem. Int., 2006, 45, 6844-6848. For the synthesis of the other compounds described in this invention, the same methodology as that already described in the above mentioned article is used.

### Synthesis of compounds 3 and 4 of the invention

The synthesis of these compounds can be seen represented in the following diagram 1:

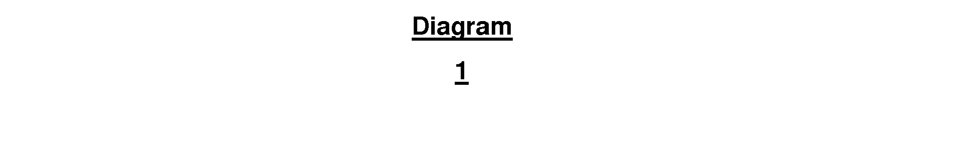

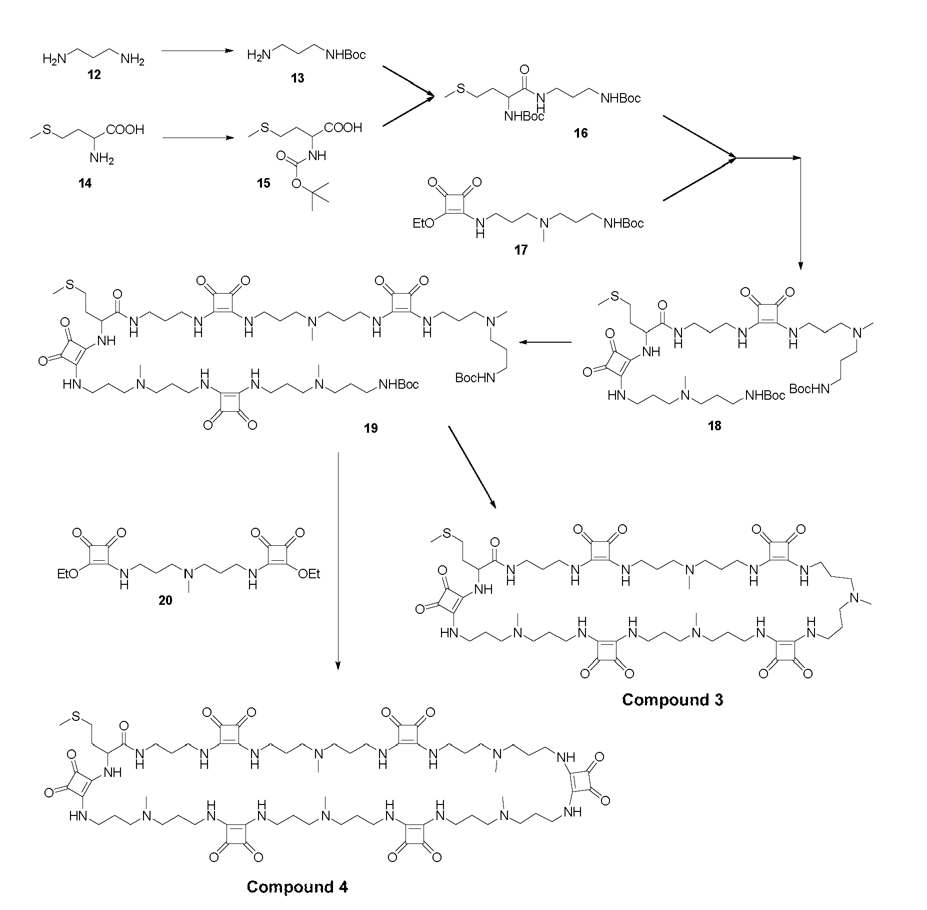

### Synthesis of compound 16

A solution of 1.076 g (5.2 mmol) of DCC in 10 ml of anhydrous CH₂Cl₂ is cooled to 0 ºC in a water-ice bath and is kept stirring and in inert atmosphere. To this solution add drop by drop 1 g (4.01 mmol) of **15** (Synthesized according to the methodology described in P. Maity, M. Zabel, B. König Journal of Organic Chemistry; 2007 72, 8046-8053), 0.7 g (4.02 mmol) of **13** (Synthesized according to the methodology described in D. J. Upadhyaya, A. Barge, R. Stefania, G.Cravotto Tetrahedron Letters 2007, 48, 8318-8322.) and 0.05 g (0.41 mmol) of DMAP in 30 ml of anhydrous CH₂Cl₂.

The mixture is kept at 0 ºC during 2 hours and it is then left for 12 h at ambient temperature. After the reaction time, it is filtered, the solvent is eliminated and the residue is suspended in Et₂O and filtered.

The product dissolved in Et₂O is precipitated by the addition of hexane obtaining 1.138 g of compound **16** as white solid, in a 70% of yield.

### Synthesis of compound 18

1.117 g (5.44 mmol) of **16** are dissolved in 6.6 ml (19.8 mmol) of 3M HCl, 30 ml of H₂O and 20 ml of MeOH. This solution is kept stirring for 2 h at 50 ºC and 12 h at 40 ºC. After this time, it is left to cool and carefully basified with solid Na₂CO₃ until pH 9-10. All the solvent is eliminated, and the residue obtained suspended in EtOH (10 ml) and filtered. The obtained solution is slowly added to a suspension of 2.41 g (5.79 mmol) of **17** (Synthesized from the methodology described in C. Rotger, M. N. Piña, M. Vega, P. Ballester, P. M. Deyà, A. Costa Anew. Chem. Int. Ed.; 2006, 45, 6844-6848) and Na₂CO₃ (20 mmol, 2.12 g) in 20 ml of EtOH. The reaction is left stirring for 24 hours, the EtOH is concentrated and the product is dissolved in CH₂Cl₂ (20 ml), washed with H₂O (2 x 10 ml), NaCl sat.(10 ml), and dried with anhydrous Na₂SO₄ and the solvent is eliminated. The resulting solid is digested in Et₂O (3 x 20 ml), obtaining 1.473 g of 18 as a pale yellow solid and in a 63% of yield.

### Synthesis of compound 19

The synthesis of **19,** is done following the same procedure as for compound **18** using 8 equivalents of hydrochloric acid. The yield of the reaction is of 65%.

### Synthesis of compound 3

0.314 g (0.24 mmol) of **19** are dissolved in 1 ml (3 mmol) of 3M HCl and 15 ml of H₂O. The solution is left at 50 ºC for 12 h. After this time, it is cooled to ambient temperature, and is basified with solid Na₂CO₃ until pH 9-10. The solvent is eliminated and the residue is suspended in 10 ml of EtOH, and is filtered. To the obtained solution, solid Na₂CO₃ (1 mmol, 106 mg)is added. Then , a solution of 0.045 g (0.26 mmol) of diethyl squarate in 1 ml of EtOH is added dropwise. The reaction is left for 24 h with stirring and at ambient temperature, the solvent is eliminated and the crude is dissolved in 2 ml of 3M HCl. The addition of NaOH until pH =9-10 allows precipitation of the product, which is separated by centrifugation and once dry is digested with CH₂Cl₂ (5 ml) obtaining 0.1 g of the compound 3 as a yellow solid in a 35% of yield. MS (ES): m/z 1198.6171 [MNa⁺]. ¹H-NMR (DMSO-d⁶, 300MHz): δ = 1.62 (br, 19H), 1.99 (s, 4H), 2.07 (s, 12H), 2.29 (br, 17H), 3.12 (br, 2H), 3.48 (br, 20H), 4.60 (br, 1H), 7.78 (br, 6H).

### Synthesis of compound 4

0.4 g (0.31 mmol) of **19** are dissolved in 1.25 ml (3.75 mmol) of 3M HCl and 20 ml of H₂O. The solution is left at 50 ºC for 12 h. After this time, it is left to cool to ambient temperature and solid Na₂CO₃ is added until to reach pH 9-10. The solvent is eliminated and the residue is suspended in 10 ml of MeOH. The suspension is filtered and the resulting solution is heated to 70 ºC for approximately 30 minutes, the heat source is removed and 0.133 g (0.34 mmol) of 20 is added dropwise (Synthesized from the methodology described in C. Rotger, M. N. Piña, M. Vega, P. Ballester, P. M. Deyà, A. Costa Angew. Chem. Int. Ed.; 2006, 45, 6844-6848*)* in MeOH (10 mL). The solution is left at ambient temperature for 24 hours, the solvent is eliminated, and the crude is dissolved 3M HCl (2 ml) and is basified with NaOH until pH = 9-10. Compound **4** precipitates, is centrifuged and dried. The solid obtained is digested with THF (2 x5 ml) and with acetone (2 x 5ml), obtaining 0.175 g of **4** as pale yellow solid and 41% of yield.
¹H-NMR (DMSO-d⁶, 300MHz): δ = 1.63 (br, 23H), 1.99 (s, 4H), 2.09 (s, 15H), 2.29 (br, 21 H), 3.12 (br, 2H), 3.48 (br, 24H), 4.61 (br, 1H), 7.42 (br, 7H). HRMS calc. for C₆₇H₁₀₇N₁₅O₁₂Na S 1198.6171; found 1198.6180 [MNa]⁺.

### 1. Kinase inhibition

The capacity of these compounds to significantly inhibit a large number of kinases "in vitro" is demonstrated, many of them of great clinical relevance. Specifically, against a panel of 210 kinases representative of the human kinome, the compounds tested inhibited by 75% the activity of 25 of them in *in Vitro* assays (Table 1).

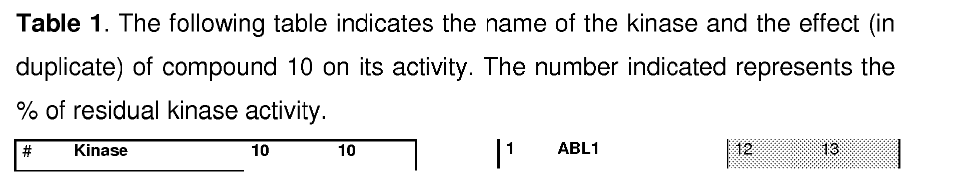

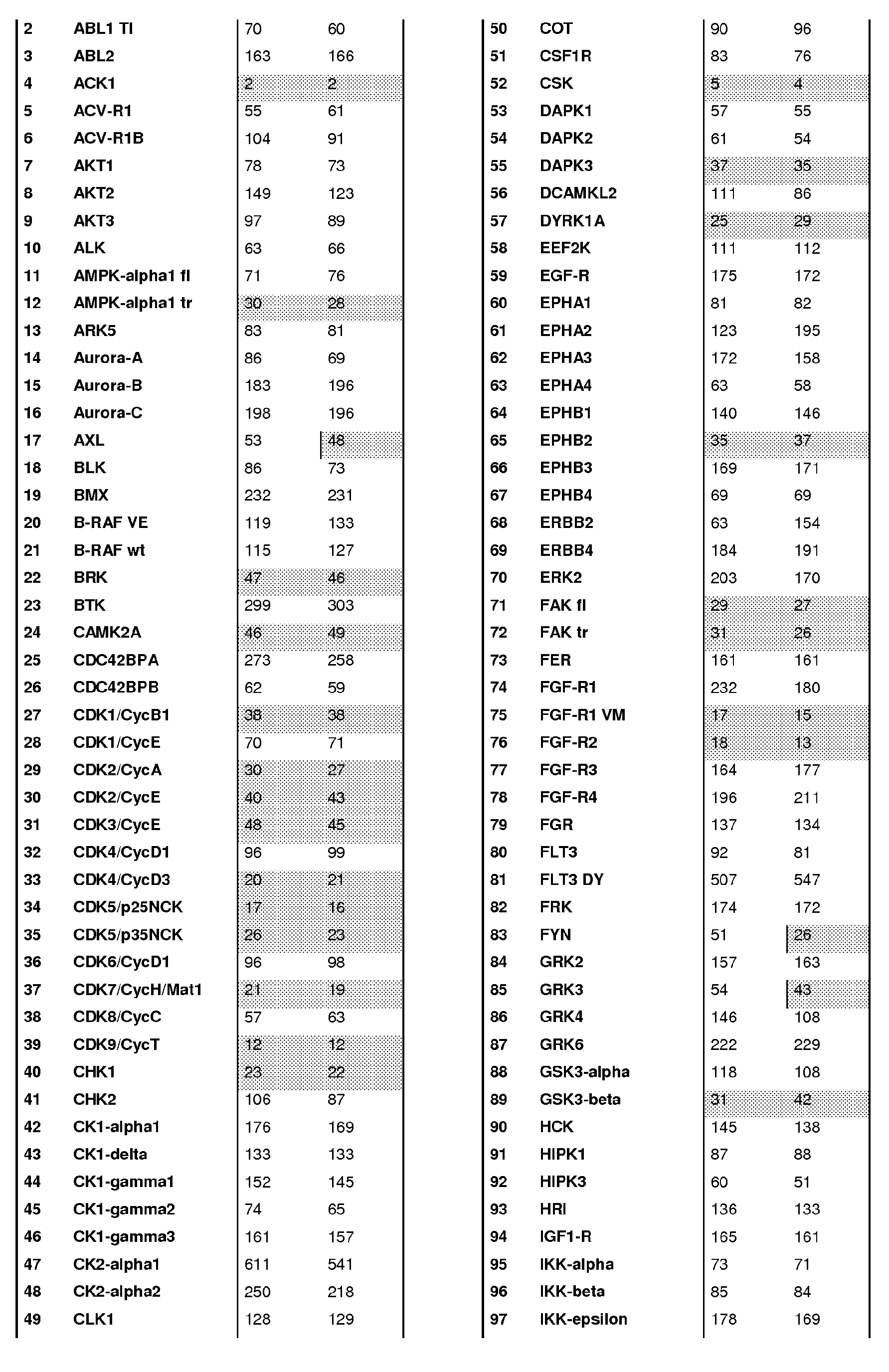

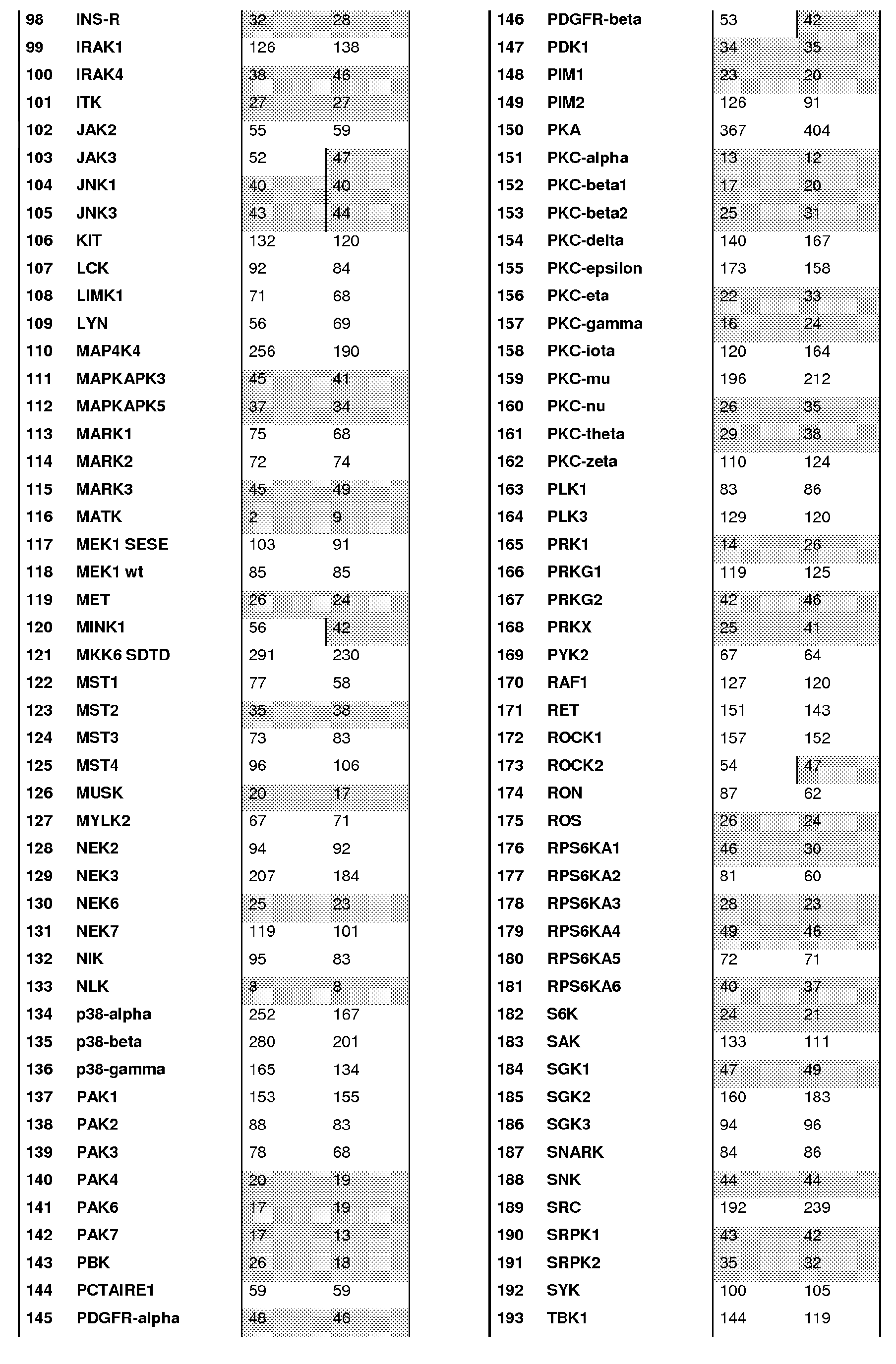

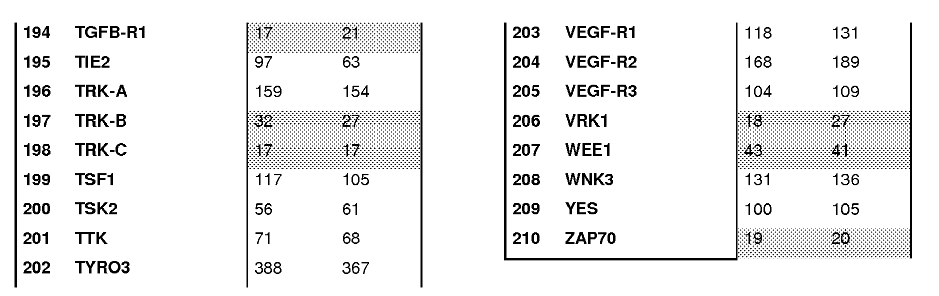

The shaded cells correspond to a residual activity ≤ 50 %

### Comparative determination of the activity of 4 compounds to 25 kinases

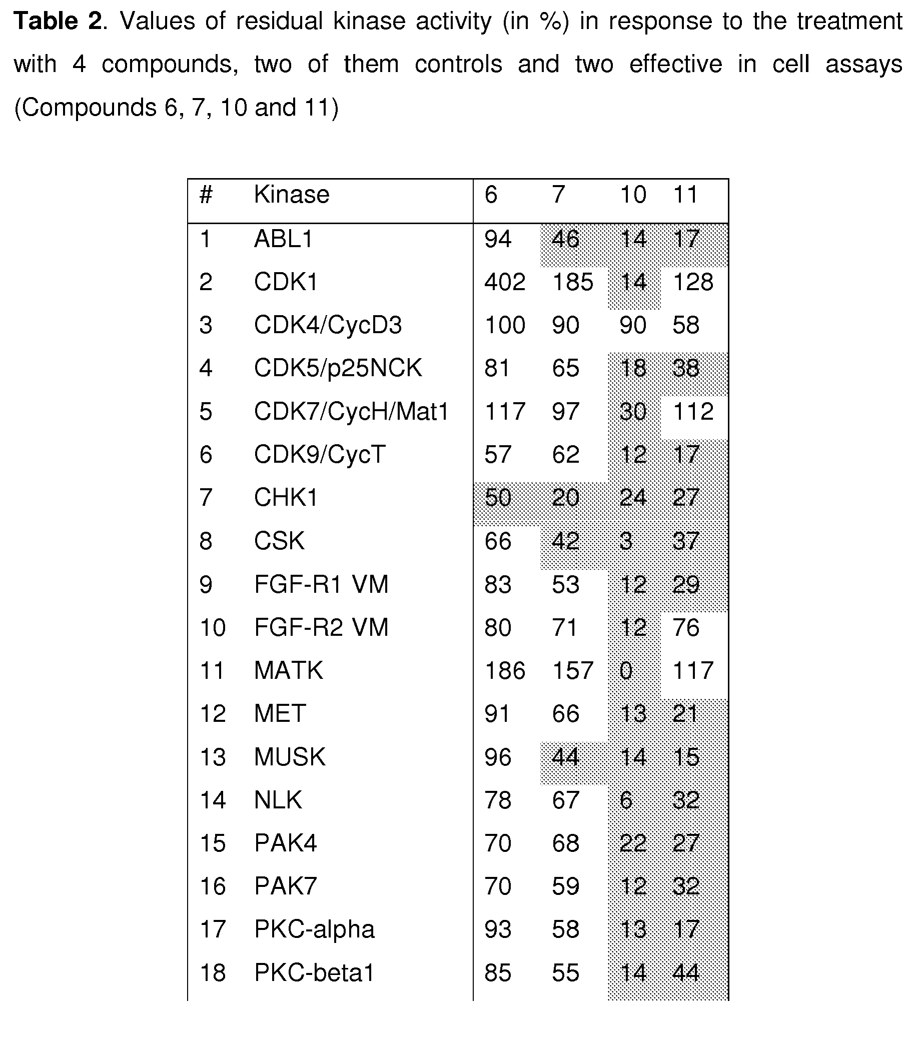

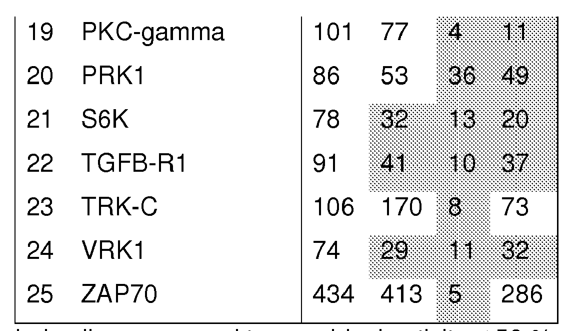

The shaded cells correspond to a residual activity ≤ 50 %

Among these kinases, there are some as relevant for tumour biology such as Abl1, cdk4, Chk1, PKC, c-Met and FGFR, among others.

### Calculation of the IC₅₀

The IC₅₀ was determined for the most sensitive kinases to compound 10, obtaining values for said parameter, largely in the micromolar range. For this purpose, the 25 kinases inhibited more than 80% by the compound in *in vitro* assays were selected. The values obtained are summarized in table 3.

**Table 3. Values of IC50 for compound 10, obtained for 25 kinases. The values of IC50 cannot be calculated for the kinases ACK1, MATK and ZAP70.**

| **No. assays** | **Kinase** | **IC50** |
|---|---|---|
| 1 | ABL1 | 5.40E-06 |
| 2 | ACK1 | - |
| 3 | CDK4/CycD3 | 5.40E-06 |
| 4 | CDK4/CycD3 | 5.30E-06 |
| 5 | CDK5/p25NCK | 9.20E-06 |
| 6 | CDK7/CycH/Mat1 | 2.80E-05 |
| 7 | CDK9/CycT | 3.70E-06 |
| 8 | CHK1 | 1.40E-06 |
| 9 | CSK | 1.30E-05 |
| 10 | CSK | 7.20E-06 |
| 11 | FGF-R1 VM | 2.60E-06 |
| 12 | FGF-R2 | 5.40E-06 |
| 13 | MATK | - |
| 14 | MATK | - |
| 15 | MET | 3.70E-06 |
| 16 | MUSK | 1.50E-06 |
| 17 | NLK | 4.30E-06 |
| 18 | NLK | 4.30E-06 |
| 19 | PAK4 | 4.30E-06 |
| 20 | PAK7 | 4.90E-06 |
| 21 | PKC-alpha | 2.30E-06 |
| 22 | PKC-beta1 | 7.60E-06 |
| 23 | PKC-gamma | 5.80E-06 |
| 24 | PKC-gamma | 4.90E-06 |
| 25 | PLK1 | 2.60E-05 |
| 26 | S6K | 4.40E-06 |
| 27 | TGFB-R1 | 3.30E-06 |
| 28 | TRK-C | 7.20E-06 |
| 29 | TRK-C | 6.60E-06 |
| 30 | VRK1 | 2.40E-06 |
| 31 | ZAP70 | - |
| 32 | ZAP70 | - |

It is interesting to highlight that the IC₅₀ determined against kinases in *in vitro* assays are very similar to the GC₅₀ obtained in the cell viability assays, suggesting that these compounds very effectively cross the cell membranes and are reasonably stable in the cytosol.

We must take into consideration the high biomedical interest in the inhibition of some of the kinases analysed. By way of example, the kinase **Ack1** (inhibited by 85% by compound 10) is clearly involved in the development of prostate cancer. The kinase **c-Met** (inhibited by 80-85% by compounds 10 and 11) is a tyrosine kinase receptor clearly involved in the development of different neoplasias, and specifically in tumour invasion. On the other hand, kinases **cdk5** and **cdk9** (inhibited by 80% and 87% by compound 10) are involved both in cancer and in various neurodegenerative diseases, in diabetes and even in HIV replication. More examples could be cited, although it is sufficient to indicate the key role of kinases inhibited by these compounds such as **S6K** or **PKC**α, among others, in cell signalling processes deregulated in numerous tumours and metabolic disease. Finally, we should recall that the kinases assessed represent a fraction of the human kinome, meaning that there are kinases not yet evaluated whose inhibition by cyclosquaramides could be greater and therefore more relevant.

### Anti-tumour assays

The antiproliferative efficacy of the oligosquaramide macrocycles has been demonstrated in cancerous cells of human lymphoma: T-cell lymphoma (Hut, Karpas-45), B-MCL lymphoma (Jeko-1, JVM-2, Rec-1, Granta-519) and glioblastoma (U-87).

As an example, the results obtained in cell viability assays are shown in table 4.

**Table 4 Summary of cell viability results (48 and 72h) after treatment with compound 10. Cell viability is represented as a percentage relative to control cells (untreated).**

| Cell line | % Cell viability |
|---|---|
| Glioblastoma | 72h |
| U87 | 25 |
| U251 | 85 |
| T-cell lymphoma | 72h |
| Hut | 60 |
| Karpas-45 | 13 |
| B-cell lymphoma (MCL) | 48h |
| Jeko-1 | 46 |
| JVM-2 | 24 |
| Rec-1 | 44 |
| Granta-519 | 58 |

### Determination of the IC₅₀ in cell viability assays

The IC₅₀ in cell viability assays were determined for some of the macrocycles in Jeko-1 cells (B-cell lymphoma) after an incubation period of 48 h.

**Table 5. IC₅₀ obtained for the compounds tested against Jeko-1 cells**

| **Compound** | **IC₅₀ (**µ**M)** |
|---|---|
| Compound 10 | 30.7 |
| Compound 11 | 9.4 |
| Compound 12 | 10 |
| Compound 5 | 50 |
| Compound 3 | 34 |
| Compound 1 | 27 |
| Compound 4 | 24 |
| Compound 2 | 45 |

As an example, growth curves are shown that are obtained in the cell viability assays to calculate the IC₅₀ against Jeko-1 cells for compounds 10 and 11 (Fig. 1 and 2).

On the other hand, the values of IC₅₀ were determined in the panel of NCI60 cell lines for compound 10.

Compound 10 generally has an antitumor activity in the order of 1 to 10 µM. It should be highlighted that some ovarian (OVCAR-4, OVCAR-5, OVCAR-8), lung (NCI-H522), colon (HT-29, HCT15), melanoma (LOX-IMVI, SK-MEL-28) and renal (786-O) cancer lines are resistance to the tested compound. However, other lines of these same types of cancer are not suggesting an interesting degree of selectivity of compound 10.

A total of 2 independent experiments were carried out with assays in triplicate for the compound and cell line. All those assays where an IC₅₀ value was not reached in the highest concentration of 1 E-05 M are represented by "> 1 E-05". In these cases, the standard deviation cannot be calculated and for that reason it is calculated as "-".

**Table 6. IC₅₀ obtained in the panel of NCI60 cell lines for compound 10.**

| **LINES** | **COMPOUND 10** | |
|---|---|---|
| | *AVERAGE* | *DESVEST* |
| SF-268 *(CNS)* | 5.61 E-06 | 0.24E-06 |
| SF-295 *(CNS)* | 5.52E-06 | 0.38E-06 |
| UO-31 *(Renal)* | 2.17E-06 | 0.08E-06 |
| DU-145 *(Prostate)* | 1.80E-06 | 0.27E-06 |
| HCT-116 *(Colon)* | 2.98E-06 | 0.03E-06 |
| IGR-OVI *(Ovarian)* | 2.38E-06 | 0.05E-06 |
| K-562 *(Leukaemia)* | 4.07E-06 | 0.05E-06 |
| MDA-MB-231 *(Breast)* | 2.32E-06 | 0.25E-06 |
| NCI-H460 *(Lung)* | 6.88E-06 | 0.42E-06 |
| PC-3 *(Prostate)* | 2.46E-06 | 0.06E-06 |
| MCF-7 *(Breast)* | 4.67E-06 | 0.10E-06 |
| Caki-1 *(Renal)* | 5.75E-06 | 0.15E-06 |
| M-14 *(Melanoma)* | 7.21 E-06 | 1.07E-06 |
| HCT-15 *(Colon)* | > 1.00E-05 | - |
| MALME-3M *(Melanoma)* | 9.28E-06 | 1.10E-06 |
| ACHN *(Renal)* | 3.25E-06 | 0.04E-06 |
| HOP-92 *(Lung)* | 3.53E-06 | 0.08E-06 |
| MDA-MB-435 S *(Breast)* | 3.66E-06 | 0.06E-06 |
| SN 12C *(Renal)* | 4.12E-06 | 0.11 E-06 |
| Sw-620 *(Colon)* | 9.41 E-06 | 0.92E-06 |

| **LINES** | **COMPOUND 10** | |
|---|---|---|
| | *AVERAGE* | *DESVEST* |
| NCI-H23 *(Lung)* | 3.02E-06 | 0.13E-06 |
| OVCAR-8 *(Ovarian)* | > 1.00E-05 | - |
| RPMI-8226 *(Leukaemia)* | 8.97E-06 | 0.55E-06 |
| DMS-114 *(Lung)* | 8.72E-06 | 0.47E-06 |
| NCI-H226 *(Lung)* | 1.53E-06 | 0.30E-06 |
| RXF-393 *(Renal)* | 2.77E-06 | 0.41 E-06 |
| TK-10 *(Renal)* | 3.36E-06 | 0.33E-06 |
| OVCAR-4 *(Ovarian)* | > 1.00E-05 | - |
| SHP-77 *(Lung)* | 2.90E-06 | 0.07E-06 |
| SK-MEL-28 *(Melanoma)* | > 1.00E-05 | - |
| Hs-578-T *(Breast)* | 7.79E-06 | 0.20E-06 |
| SW-480 *(Colon)* | 1.80E-06 | 0.09E-06 |
| DAUDI *(Leukaemia)* | 1.99E-06 | 0.66E-06 |
| MOLT-4 *(Leukaemia)* | 1.36E-06 | 0.35E-06 |
| NCI-H522 *(Lung)* | > 1.00E-05 | - |
| SK-BR-3 *(Breast)* | 3.08E-07 | 0.19E-06 |
| T47-D *(Breast)* | 1.89E-06 | 0.61 E-06 |
| OVCAR-3 *(Ovarian)* | 5.07E-06 | 0.26E-06 |
| SF539 *(Astrocytoma)* | 3.52E-06 | 0.81 E-06 |
| ADR-RE-3 *(Breast)* | 1.62E-06 | 0.46E-06 |
| | | |

| **LINES** | **COMPOUND 10** | |
|---|---|---|
| | *AVERAGE* | *DESVEST* |
| 786-O *(Renal)* | > 1.00E-05 | - |
| CCRF-CEM *(Leukaemia)* | 2.09E-06 | 0.20E-06 |
| Hl-60 *(Leukaemia)* | 2.41 E-06 | 0.08E-06 |
| MDA-MB-468 *(Breast)* | 2.37E-06 | 0.03E-06 |
| NCI-H522 *(Lung)* | 1.63E-06 | 0.17E-06 |
| OVCAR-5 *(Ovarian)* | > 1.00E-05 | - |
| A-498 *(Renal)* | 7.50E-06 | 0.48E-06 |
| BT-549 *(Breast)* | 2.21 E-06 | 0.10E-06 |
| COLO-205 *(Colon)* | 3.23E-06 | 0.06E-06 |
| EKVX *(Lung)* | 2.75E-06 | 0.05E-06 |
| HT-29 *(Colon)* | > 1.00E-05 | - |
| LOX-IMVI *(Melanoma)* | > 1.00E-05 | - |
| SK-MEL-2 *(Melanoma)* | 3.29E-06 | 0.03E-06 |
| SK-MEL-5 *(Melanoma)* | 2.75E-06 | 0.00E-06 |
| SNB-19 *(CNS)* | 1.81 E-06 | 0.19E-06 |
| A549 *(Lung)* | 5.29E-06 | 0.04E-06 |
| HOP-62 *(Lung)* | 6.37E-06 | 0.40E-06 |
| SK-OV-3 *(Ovarian)* | 3.10E-06 | 0.04E-06 |
| U-251 *(CNS)* | 7.86E-06 | 0.37E-06 |
| UACC-62 *(Melanoma)* | 1.34E-06 | 0.06E-06 |

The macrocyclic oligosquaramides exert cytostatic and cytotoxic effects on the various cell models of untested human tumour cells.

### Studies of the cell cycle and apoptosis profile by flow cytometry

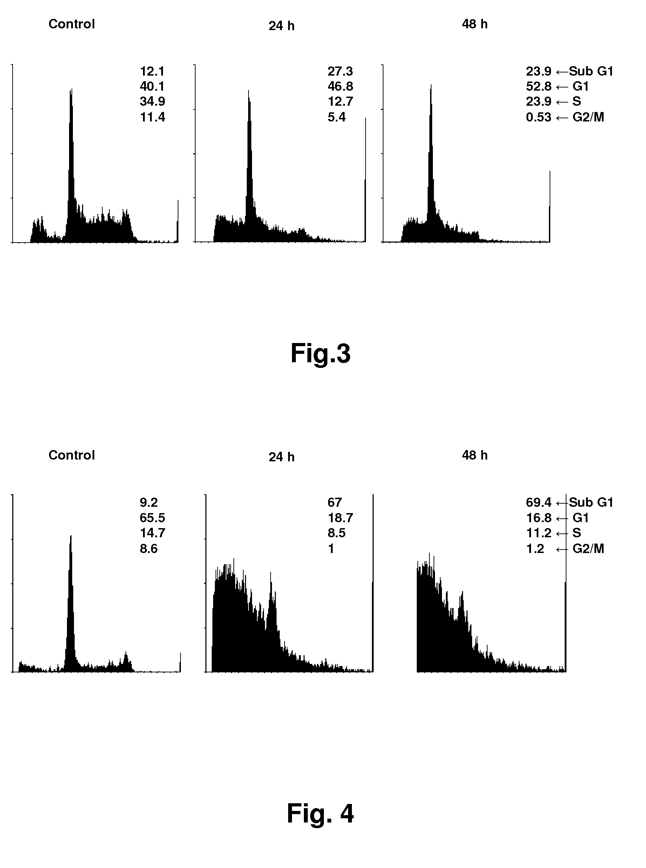

It has been proven by flow cytometry that the macrocyclic oligosquaramides tested induce, depending on the cell line, the inhibition of G1-phase progression and/or the induction of apoptosis (sub-G1 cells) (see Fig. 3 and 4).

### Multicellular spheroid growth assays

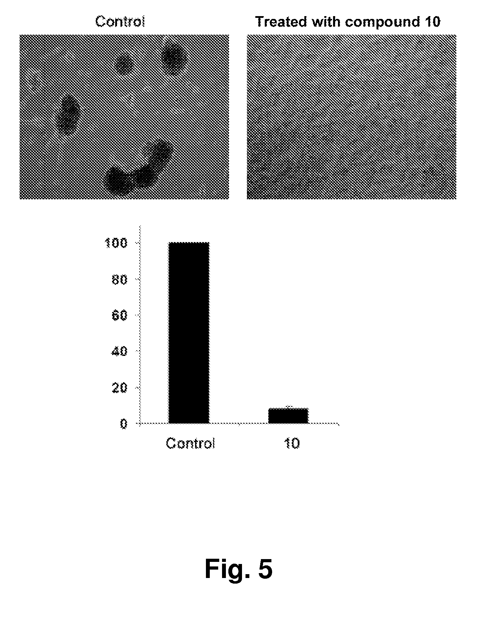

In the Glioblastoma U87 cells, these compounds reduce their growth and viability in the long-term and in 3D, by inhibiting the formation of multicellular tumour spheroids and by reducing their clonogenic capacity (see Fig. 5).

### Toxicity and Selectivity

Furthermore, it is especially interesting to highlight that these compounds do not act as indiscriminate cell toxins but they show a high selectivity towards some cell types. Indeed, they do not affect the viability of normal cells such as NIH 3T3. This selectivity is even shown against tumour cell lines of the same origin: for example, whilst they are very active in glioblastoma U87 cells, they practically do not affect U251 cells, also of glioblastoma. Similarly, they show must greater activity to some subtypes of lymphoma cells than others. All this suggests that they act as specific cell targets of some cell types, in correlation with their activity against numerous kinases.

## Claims

1. Use of the compound of general formula (I): 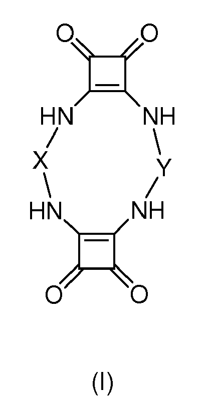 where:
X and Y, are the same or different and selected from the list comprising:
- a alkyl group (C₂-C₉);
- a -R¹-NR-R²- group; or
- a-(R¹-NR-R²-squaramide-R¹-NR- R²)n group; where
R is selected from the alkyl (C₁-C₅), aryl or heteroaryl groups, substituted or non-substituted;
R¹ and R², are the same or different and are selected from the alkyl (C₁-C₅) or acyl groups, substituted or non-substituted; and
n takes the values of between 1 and 8.
or any of their salts, for the preparation of a pharmaceutical composition.

2. Use of the compound according to claim 1, wherein X or Y is a heptyl group.

3. Use of the compound according to any of claims 1 or 2, wherein X or y is a -R¹-NR-R²- group.

4. Use of the compound according to claim 3, wherein R¹ or R² is a propyl group.

5. Use of the compound according to claim 3, wherein R¹ or R² is an acyl group (COR³), and R³ is an alkyl group substituted with a thioalkyl group (C₁-C₃).

6. Use of the compound according to any of claims 1 to 5, wherein X and Y are a -(R¹-NR- R²-squaramide- R¹-NR- R²)n group, where n takes the values of 1 to 6.

7. Use of the compound according to any of claims 1 to 6, wherein R is a methyl, benzyl or substituted benzyl group.

8. Use of the compound according to claim 7, wherein R is a nitrobenzyl group.

9. Use of the compound according to claim 1, of formula: 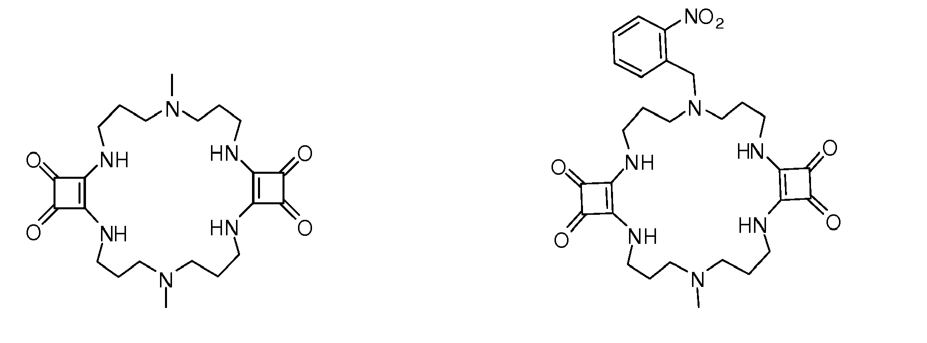 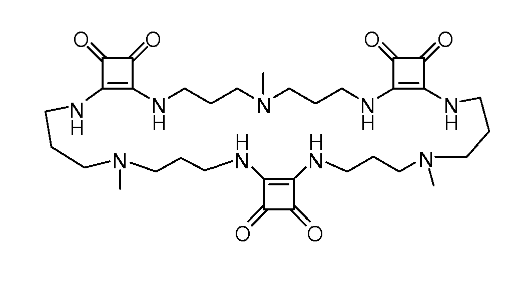 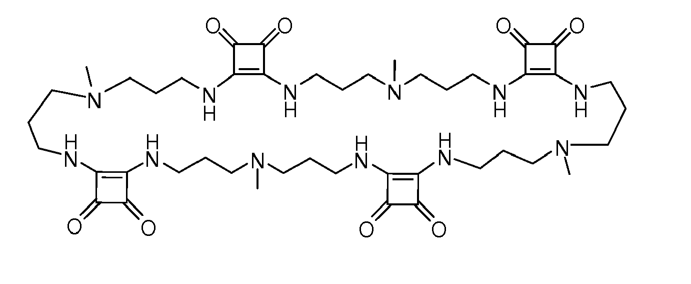 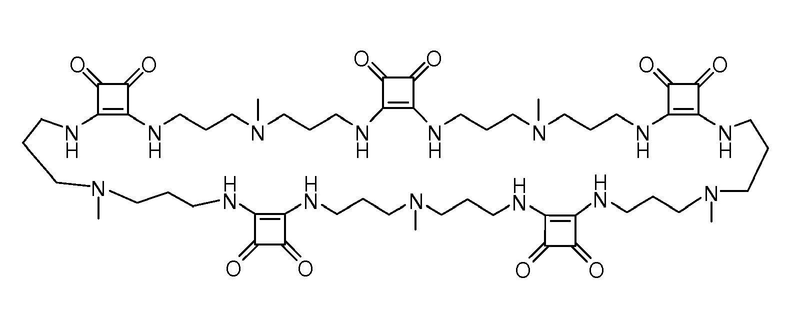 or

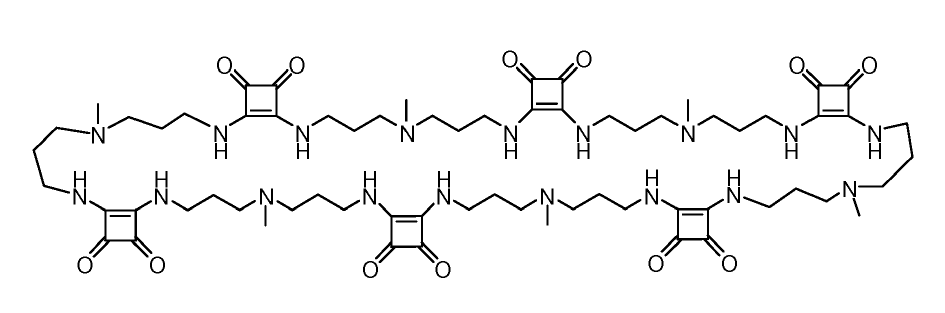

10. Use of the compound according to any of claims 1 to 9, for the treatment of diseases associated with kinase inhibition.

11. Use of the compound according to claim 10, wherein the diseases associated with kinase inhibition are tumoral diseases.

12. Use of the compound according to claim 11, wherein the tumours are B and T-cell lymphoma or glioblastoma.

13. Use of the compound according to claim 10, wherein the diseases associated with kinase inhibition is diabetes.

14. Use of the compound according to claim 10, wherein the diseases associated with kinase inhibition are neurodegenerative diseases.

15. Use of the compounds according to claim 14, wherein the neurodegenerative disease is Alzheimer.

16. Use of the compound according to claim 10, wherein the diseases associated with kinase inhibition is HIV replication.

17. Pharmaceutical composition comprising at least one compound of general formula (I) and a pharmaceutically acceptable vehicle.

18. Pharmaceutical composition according to claim 17 which further comprises another active principle.

19. Compound selected from the list comprising: 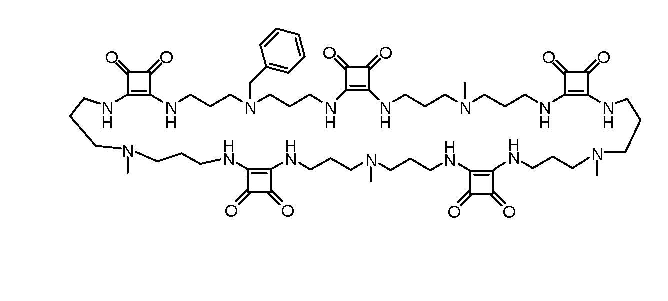 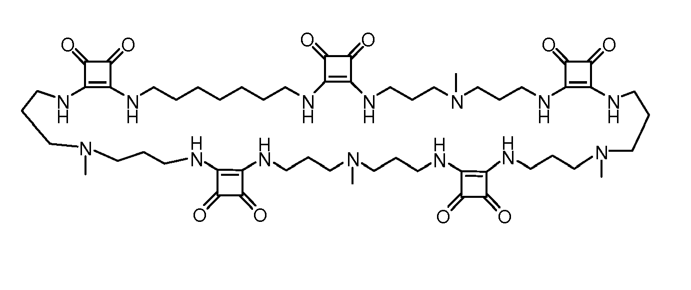 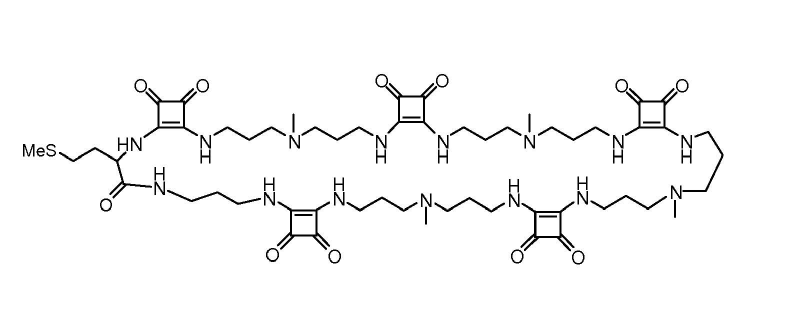 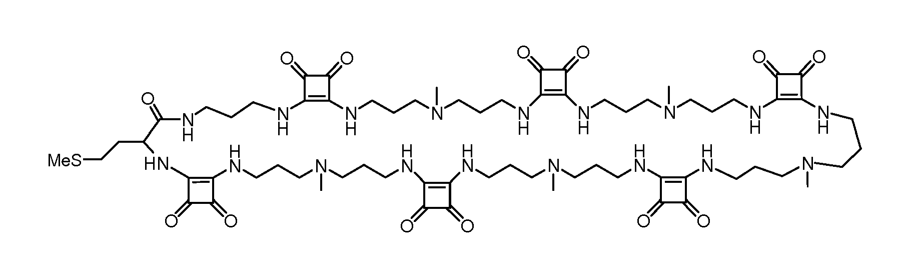 or
